# EUROPEAN PATENT APPLICATION

(11) **EP 1 939 212 A1**
(43) Date of publication of application: **02.07.2008**
(21) Application number: 06126727.4
(22) Date of filing: 20.12.2006
(51) Int. Cl.: C07K 1/14, C07K 1/36, C12N 1/06, C12P 21/00

(54) **Organic compounds**

(71) Applicant: LEK Pharmaceuticals d.d., 1526 Ljubljana (SI)
(72) Inventor: The designation of the inventor has not yet been filed
(74) Representative: Lehmeier, Thomas Jürgen

(57) **Abstract**

The present invention relates to a new process for the production of a polypeptide of interest, or of a precursor thereof, wherein the protein or the precursor are expressed in a host cell comprising a cell wall and at least one cell membrane, followed by extraction from the intact host cell by partial permeabilization of the cell membrane under non-denaturing conditions. During extraction the polypeptide of interest, or the precursor thereof, which is expressed in at least partially correctly folded form, passes through the cell wall and the cell membrane.

## Description

### Field of invention

The present invention relates to a new process for the production of a polypeptide of interest, or of a precursor thereof, wherein the protein or the precursor are expressed in a host cell comprising a cell wall and at least one cell membrane, followed by extraction from the intact host cell by partial permeabilization of the cell membrane under non-denaturing conditions. During extraction the polypeptide of interest, or the precursor thereof, which is expressed in at least partially correctly folded form, passes through the cell wall and the cell membrane. The principle of the present invention can be applied to a wide range of heterologous and homologous proteins as well as fusion proteins expressed in a variety of host cells, e.g. in E. coli. Exemplarily the process is described by referring to the process for the production of Green Fluorescent Protein (GFP) and human Granulocyte-Colony Stimulating Factor (hG-CSF).

### Background of invention

The isolation of the expressed protein from a host cell is a crucial step in the production of proteins. Depending on the host organism, the very polypeptide of interest and the culture conditions, the polypeptide of interest can be accumulated within the cell in different forms. A polypeptide of interest may be accumulated in soluble form within the cytoplasm of the host cell, it may be transported into the periplasmic space, or it may be accumulated inside of the cell in so called "inclusion bodies". Only in rare cases export of the polypeptide of interest into the bacterial periplasm can be induced with the aid of a pro- or eukaryotic signal sequence. Because of the low transport capacity of the bacterial export machinery usually only very small quantities of product can be accumulated in this way. The way of deposition of the protein within the host cell determines how it can be retrieved and which further processing is required before the protein can be subjected to purification.

The extraction of proteins of interest from host cells is generally started by disruption of the host cell. This can be achieved e.g. by mechanical disruption or by enzymatic lysis of the cells. Classical mechanical disruption requires the use of high-pressure homogenizers.

Disruption must be performed in a closed system to avoid the release of spray which contains bacterial endotoxins into the surrounding.

If enzymatic cell disruption, e.g. by lysozyme, is applied, the cell membranes and the cell walls of the cells are demolished by enzymatic lysis. This way, a comparable result as with the mechanical cell disruption is achieved.

Following the disruption of the cells, the remaining cell fragments must be separated from the liquid phase of the suspension. This is generally achieved by centrifugation, which has to be performed in a closed system as well, due to the spray of bacterial endotoxins.

During cell disruption usually the whole content of the cell including a high amount of homologous proteins and chromosomal DNA is released from the host cell. This is another drawback of cell disruption since these proteins and DNA must be removed from the protein extract in the following further downstream processing.

Proteins which are expressed soluble within the cell are generally separated from the cell debris by microfiltration or centrifugation. The resulting protein solution then needs to be subjected to further purification steps, which usually include a sequence of chromatographic purification steps.

If a polypeptide of interest is over expressed in bacterial cells, especially when strong promoters are used, often high density polypeptide aggregates are formed, which are deposited in the cytoplasm of the cell as so called inclusion bodies. The production of polypeptides in the form of inclusion bodies is of special interest for production on industrial scale, since the expressed polypeptide is present in high amounts in the inclusion bodies. Also, proteins deposited in inclusion bodies are protected from cellular proteases. The fact that further work up is required after isolation of cytoplasmatic inclusion bodies from the host cells presents a major drawback of production of polypeptides in this form. Such work up usually involves e.g. solubilization of the inclusion bodies using high concentration of denaturants, followed by a renaturation step.

Typically the preparation of proteins from inclusion bodies starts with a washing step. The washing solution usually contains a detergent, e.g. Deoxycholate, or Triton X100. Washing is followed by solubilization of the inclusion bodies. Due to the low solubility of the inclusion bodies this step generally requires the use of strong denaturants, e.g. high concentrations of urea or guanidium chloride. Solubilization of the inclusion bodies is followed by in vitro renaturation. This step requires large process volumes. Thus processing of inclusion bodies is complex, especially since correct refolding is required during the renaturation step in order to gain the biologically active form of the polypeptide of interest.

In view of the tedious processes described above, there is a strong need for a simplified process of protein production, especially in respect of protein extraction and preparation of proteins from inclusion bodies.

Within the present invention it was surprisingly found that it is possible to extract a polypeptide of interest from inside the host cell without cell disruption if, for a host cell which comprises a cell wall in addition to at least one cell membrane, the cell membrane is partially permeabilized in such a way, that certain molecules can diffuse through the partially permeabilized membranes, while the mechanical stability of the host cell remains intact. This is achieved by incubating the host cell in mild detergents under non-denaturing conditions. Following the partial permeabilization the polypeptide of interest is extracted from the cell and accumulated in the liquid part of the cell suspension. Compared to classical processes of protein extraction, which require the disruption of the host cell, the process of the present invention provides a more selective way of extraction of the polypeptide of interest, during which also less host cell DNA is released from the cell. In addition the cells are not fragmented and can easily be separated from the protein solution either by centrifugation or by expanded bed adsorption techniques. If the polypeptide of interest is expressed by the host cell in form of classical or non-classical inclusion bodies, in addition to cell disruption also the steps of de- and renaturation become unnecessary. In this case moreover, since the extraction is carried out under non-denaturing conditions, after extraction the polypeptide of interest is soluble and in certain cases also biologically active. Thus, if expression of the polypeptide of interest in form of non-classical inclusion bodies is combined with the process of the present invention, the benefits of expression of proteins in form of inclusion bodies can be exploited, without the usually required difficult, time-consuming, inefficient and costly extraction, denaturation and renaturation steps.

### Summary of the Invention

The present invention relates to a new process for the production of a heterologous or homologous polypeptide of interest, wherein the polypeptide of interest or a precursor thereof is expressed in at least partially correctly folded form within a host cell, which comprises a cell wall and at least one cell membrane. After expression the polypeptide of interest is extracted from said host cell by partially permeabilizing the cell membrane under non-denaturing conditions using mild detergents. This partial permeabilization of the cell membrane allows diffusion of certain molecules e.g. the polypeptide of interest, through the cell membrane, while the cell, supported by its cell wall, remains intact. Thus the polypeptide of interest is accumulated in the liquid part of the cell suspension and can easily be separated from the intact cell, e.g. either by centrifugation or expanded bed adsorption techniques.

### Detailed description of the invention

The present invention is related to a process for the recombinant production of a polypeptide of interest within a host cell which comprises a cell wall and at least one cell membrane, comprising:
(i) cultivation of said host cell under conditions which cause expression of the polypeptide of interest or of a precursor of the polypeptide of interest in at least partially correctly folded form within said host cell and
(ii) extraction of the polypeptide of interest or the precursor of the polypeptide of interest from said host cell under non-denaturing conditions, whereby the cell membrane is partially permeabilized while the cell remains intact and whereby the polypeptide of interest is extracted through the cell membrane and the cell wall.

Within the present invention it has surprisingly been found that a heterologous or homologous polypeptide of interest or a precursor of said protein, expressed within a host cell which comprises a cell wall and at least one cell membrane in at least partially correctly folded form (in soluble form or in form of classical or non-classical inclusion bodies) can be isolated from the host cell by the use of mild detergents under non-denaturing conditions without prior cell disruption.

Accordingly the present invention preferably refers to a process, wherein the extraction of the polypeptide of interest is performed using a mild detergent.

More preferably the present invention refers to a process, wherein the extraction of the polypeptide of interest is performed using NLS.

Most preferably the extraction according to the present invention is performed using NLS in a concentration of between 0.05% and 0.2%.

Accordingly the present invention refers also to the use of NLS in the extraction according to the present invention.

The term "detergent" as used herein refers to any ionic or non-ionic substance or mixtures of such substances able to form micelles.

The term "mild detergent" as used herein refers to a detergent which, while enabling permeabilization of the host cell membranes, keeps the cell intact without affecting the biological activity of the polypeptide of interest. For each host cell and polypeptide of interest the suitable concentration for a particular detergent is determined experimentally. A person skilled in the art can determine the suitable concentration without undue burden.

The term "intact cell" as used herein refers to a cell that is mechanically intact, not disrupted, nor collapsed or burst.

The suitable concentration of a particular detergent can be determined by incubation of the pure biologically active polypeptide of interest in solutions comprising various concentrations of that detergent under the same conditions (e.g. temperature, buffering system, pH) that are chosen for the extraction. The biological activity of the polypeptide of interest is assessed for each solution comprising different concentrations of said detergent. Tests for biological activity of proteins are generally known. One such method is described in example 8.

The term "denaturation", as used herein, refers to a process in which the native conformation of the protein (three dimensional structure) is changed, but the primary structure (amino acid chain, peptide links) of the protein remains unchanged. Upon denaturation the protein looses its biological activity.

The term "renaturation", as used herein, refers to a process in which, starting from a denatured protein, the native conformation of the protein (three dimensional structure) is restored. Upon renaturation the protein regains its biological activity.

The term "non-denaturing conditions", as used herein, refers to conditions, in which the native conformation of the protein, its three dimensional structure, is maintained. The biological activity of a protein is preserved under such conditions.

The term "partial permeabilization", as used herein, refers to the removal of some membrane proteins from the host cell. Thus certain molecules can pass through the cell membrane, while the cell remains intact. E.g. water, ions, extraction substances can pass from the outside into the cell, while molecules present inside of the cell, e.g. proteins, can pass in the opposite direction.

The term "heterologous protein", as used herein, refers to a protein which is foreign to the host organism in which it is expressed.

The term "homologous", as used herein, refers to a protein which is naturally expressed by the host organism.

Within one preferred embodiment of the present invention the polypeptide of interest is expressed soluble in the cytoplasm of the host cell.

Within another equally preferred embodiment of the present invention the polypeptide of interest is expressed within the host cell in the form of non-classical inclusion bodies.

Within yet another equally preferred embodiment of the present invention the polypeptide of interest is expressed within the host cell in the form of classical inclusion bodies.

The term "inclusion bodies" is generally understood to denote compact aggregates of incorrectly folded or partially correctly folded proteins which are highly insoluble so that strong denaturants are needed for solubilization. These inclusion bodies are referred to as "classical inclusion bodies" in the following.

The term "non-classical inclusion bodies" as used herein, refers to inclusion bodies which are characterized by their greater solubility and higher content of correctly folded protein and/or correctly folded precursor of the polypeptide of interest, when compared to classical inclusion bodies. These non-classical inclusion bodies are soluble under non-denaturing conditions, e.g. in non-denaturing aqueous solutions. Non-classical inclusion bodies are formed under certain specific fermentation conditions, as described in S. Jevsevar et al. (2005), Biotechnolog. Prog. 21, 632-639 which is herewith incorporated by reference. It has been found that by affecting the cultivation or fermentation of the host organism, especially by affecting the performed biosynthesis, the production of proteins of interest, heterologous and homologous, can be adjusted in such a way that a polypeptide of interest is accumulated in non-classical inclusion bodies. Special requirements for work up of proteins expressed in such non-classical inclusion bodies are also described in S. Jevsevar et al. (2005), Biotechnolog. Prog. 21, 632-639, which is incorporated by reference.

The term "precursor" of the polypeptide of interest as used herein, refers to a protein (e.g. G-CSF) having the correct conformation (the native conformation may be present or inherently generated), wherein the disulfide bridges may not be formed or not yet be formed completely. After extraction of the precursor from the host cell the intramolecular disulfide bridges form either spontaneously or their formation is induced by addition of a catalyst, such as a metallic salt, e.g. CuSO₄, to the washing solution. Such "precursor" of the polypeptide of interest is present in non-classical inclusion bodies. It can be directly solubilized without denaturants or strong alkaline solutions and is capable of providing a biologically active protein.

The process of the present invention can be used for proteins expressed in any host organism, provided that it has a cell wall and at least one cell membrane. Suitable host organisms can be e.g. bacteria or yeasts. From suitable bacterial hosts E. coli and Streptomyces sp., Bacillus sp. are preferred, E. coli is a particularly preferred host organism. Of potential yeast host organisms e.g. Saccharomyces cerevisiae or Pichia pastoris, Hansenula polymorpha, or Candida utilis can be employed.

Accordingly the present invention relates to a process for the production of a polypeptide of interest, wherein the polypeptide of interest is expressed in a bacterial or a yeast host cell.

In a more preferred embodiment of the present invention, the polypeptide of interest is expressed in a bacterial host cell.

Even more preferred is an embodiment of the present invention, wherein the polypeptide of interest is expressed in an E. coli host cell.

Suitable host cells are transformed with plasmids in a manner known per se, in order to express the respective polypeptide of interest. Fermentation conditions known in the art are chosen and the process is adjusted in such a way, that the respective polypeptide of interest or a precursor thereof is expressed in an at least partially correctly folded form. Preferably fermentation conditions are chosen, that result in protein expression soluble within the cytoplasm of the cell, or in form of classical or non-classical inclusion bodies.

For the process of the present invention, after fermentation, the cells are centrifuged in a manner known in the art and the supernatant is discarded. The cell pellet is re-suspended in extraction solution.

According to the present invention the cells may be incubated in extraction solution for a certain period. Following this incubation the cells are centrifuged and the supernatant is collected. This sequence of steps is referred to in the following as an "extraction cycle". The remaining cell pellet may be re-suspended in fresh extraction solution, while the supernatant can be collected for further processing. The whole extraction can be repeated in identical cycles as described above, while the supernatant is collected after each incubation cycle.

According to the present invention, a single extraction cycle may be sufficient for extraction of the polypeptide of interest.

Preferably the extraction according to the present invention is performed by a single extraction cycle or, equally preferably, by a series of consecutive extraction cycles.

Most preferably the extraction is performed by a series of between 2 and 4 consecutive extraction cycles.

According to the present invention the duration of the extraction cycles may be between 1 and 20 hours, preferably it is between 3 and 5 or, equally preferably, between 8 and 10 hours.

Thus according to the present invention the extraction may also be performed as a two-step procedure, wherein in a first step the cells are incubated in a permeabilization solution followed by extraction in a second step as described above. Also after partial permeabilization extraction can be performed either by a single extraction cycle or by repeated cycles, as described above. According to the present invention prior to extraction the host cell may be partially permeabilized by incubation in a solution comprising a mild detergent, e.g. NLS (N-lauroyl sarcosine).

In a preferred embodiment of the present invention, prior to extraction, the host cell is subjected to a partial permeabilization step.

In a more preferred embodiment of the present invention the host cell is partially permeabilized by incubation in a solution comprising a mild detergent.

In an even more preferred embodiment, the mild detergent is NLS.

Preferably the concentration of NLS for partial permeabilization is between 0.05% and 0.2%. Most preferably the concentration of NLS is 0.1 %.

Further, the present invention provides a process for the extraction of a biologically active polypeptide of interest from classical or non-classical inclusion bodies, while these inclusion bodies are still inside the cell.

Surprisingly it has also been found within the present invention, that in the case of proteins being expressed in the form of classical or non-classical inclusion bodies, the extraction according to the present invention is accelerated by the presence of water soluble, small, polar molecules in the extraction solution. Such molecules are referred to herein as "extraction substances". One example of such an "extraction substance" is NDSB (non-detergent sulfobetaine 201).

According to the present invention extraction substances can also be used for the extraction of proteins of interest which are present in the host cell in soluble form.

The term "extraction substance" as used herewith refers to water soluble, small, polar molecules, which do not permeabilize the cell-membranes but enhance the dissolution of a polypeptide of interest which is deposited within the cell e.g. in form of classical or non-classical inclusion bodies or in soluble form, under non-denaturing conditions.

In another aspect of the present invention, the extraction of the polypeptide of interest is performed using an extraction solution comprising a mild detergent and in addition an extraction substance.

More preferred is a process according to the present invention, wherein the mild detergent is NLS and the extraction substance is NDSB.

Again more preferred is a process according to the present invention, wherein NLS is used in a concentration of between 0.05% and 0.1 %, and NDSB is used in a concentration of between 0.2% and 2%.

Most preferred is a process, wherein NLS is used in a concentration of 0.1 %, and NDSB is used in a concentration of 2%.

In another preferred embodiment of the present invention, extraction solutions comprising only a mild detergent (e.g. NLS) and extraction solutions comprising an extraction substance (e.g. NDSB) in addition to a mild detergent (e.g. NLS) are applied in alternating extraction cycles.

In another, preferred embodiment of the present invention, the partial permeabilization is performed for about 30 minutes in a solution comprising about 0,05% - 0,2%NLS, while the subsequent extraction steps are performed in an extraction solution comprising about 0,2-2% NDSB.

If the host cell has been partially permeabilized, according to the present invention, extraction may be performed in an extraction solution comprising only an extraction substance, and no mild detergent.

In a preferred embodiment of the present invention the extraction from a partially permeabilized host cell is performed using an extraction solution comprising an extraction substance and no mild detergent.

More preferably the extraction solution comprises NDSB and no mild detergent.

Particularly preferably the extraction solution comprises NDSB in a concentration between 0.2% and 2% and no mild detergent.

According to the present invention an extraction solution comprising an extraction substance (e.g. NDSB) in addition to a mild detergent (e.g. NLS) may be used for the extraction of proteins that are expressed in form of classical or non-classical inclusion bodies, or soluble within the cell, e.g. G-CSF, GFP or IFN-alpha.

Also according to the present invention an extraction solution comprising only an extraction substance (e.g. NDSB) and no mild detergent may be used for the extraction of proteins that are expressed in form of classical or non-classical inclusion bodies, or soluble within the cell, e.g. G-CSF, GFP or IFN-alpha.

Alternatively according to the present invention an extraction solution comprising only a mild detergent (e.g. NLS) and no extraction substance may be used for the extraction of proteins that are expressed in form of non-classical inclusion bodies or soluble within the cell. This process can be employed e.g. for the extraction of G-CSF, GFP or IFN-alpha.

Particularly preferred is an embodiment of the present invention, wherein the polypeptide of interest is G-CSF.

The present invention further relates to the purification of a protein, produced by the process according to the present invention.

The present invention also refers to the use of NLS as mild detergent for the partial permeabilization of a cell membrane of a host cell within the process of the present invention.

The present invention refers as well to the use of NDSB as an extraction substance within the process of the present invention.

All extraction cycles may be run at a temperature of about 2°C to about 25°C, more preferably at a temperature between about 4°C and 20 °C, 4°C being most preferred.

The pH of the extraction solution is adjusted depending on the respective protein to be extracted. The aim is to maximally stabilize the polypeptide of interest. It is known to a person skilled in the art how to select, adjust and maintain the optimal pH for each polypeptide of interest.

The solution comprising the extraction substance may be buffered, but depending on the stability of the respective polypeptide of interest un-buffered solutions can also be applied.

The process disclosed in the present invention results in a cell suspension comprising the extraction solution, the intact host cells and the polypeptide of interest or its precursor. This cell suspension can directly be subjected to the first step in downstream processing, e.g. expanded bed adsorption (EBA). Alternatively the cells can be removed from the protein solution by centrifugation and the solution can then be applied to further purification steps, e.g. to a first chromatographic step (capture phase).

Also, according to the present invention, the extraction procedure can be coupled with expanded bed adsorption (EBA). As the target protein is continuously removed from the extraction solution by adsorbing to the EBA matrix, a faster extraction is achieved.

The process of the present invention can be employed for the production of a wide range of pharmaceutically active proteins, e.g. for the production of interferons (IFN), such as IFN-alpha, IFN-beta, IFN gamma, interleukins (IL), such as IL-2 and IL-4, granulocyte macrophage colony stimulating factor (GM-CSF), macrophage stimulating factor, (M-CSF), epidermal growth factor (EGF), human serum albumin (HAS), deoxyribonuclease (DNAse), fibroblast growth factor (FGF); tumor necrosis factor alpha (TNF alpha) and tumor necrosis factor beta (TNF beta), fragments of antibodies, e.g., Fab fragments. Further examples for proteins that can be produced by the process of the present invention comprise proteins without pharmaceutical activity e.g. GFP, and fusion proteins of either pharmaceutically active or pharmaceutically non-active proteins.

The present invention is described further with reference to the following examples, which are illustrative only and non-limiting. In particular, the examples relate to preferred embodiments of the present invention.

### Examples

Compositions of buffers used in examples
PB1: 0,1 % NLS in 20 mM TRIS/HCl, pH 8.0
PB2: 0,2 % NLS in 40 mM TRIS/HCl pH 8.0
PB3: 0.05 % NLS in 40 mM TRIS/HCl, pH 7.5-8.0
EB1: 0,2 % NLS in 40 mM TRIS/HCl, pH 8.0
EB2: 2 % NLS in 40 mM TRIS/HCl, pH 8.0
EB3: 0.2 % NDSB-201 in 40 mM TRIS/HCl pH 8.0
EB4: 2 % NDSB-201 in 40 mM TRIS/HCl pH 8.0
EB5: 0.05 - 0.1 % NLS; 1-2 % NDSB-201; 20-50 mM Tris/HCl, pH 7.5-8,0

### Example 1: Expression of the polypeptide of interest

The preparation as well as the cloning of the gene for G-CSF, the Fopt5 gene, into a pET9a plasmid is described in detail in WO 2004/013175 A1, which is hereby incorporated by reference. The same procedures are used for cloning of Fopt5 into the pET9a plasmid, which is used in the following examples. The pET9a plasmid is described in Makrides 1996, table 1 page 514.

Biosynthesis is performed in a 5 litre laboratory fermentor at 25-30° C using the production strain E. coli BL21 (DE3)-pET9a-Fopt5. Most of G-CSF is expressed in form of non-classical inclusion bodies. The host cells are centrifuged and the supernatant is discarded.

### Example 2: Two step extraction of G-CSF from partially permeabilized E. coli cells using NLS

A two-step extraction is applied. In step 1 the wet bacterial pellet is re-suspended in 5 volumes of a permeabilization buffer (PB1 and aliquoted (approximately 1 gram of wet pellet per aliquot). After 30 minutes of incubation (permeabilization) the suspension is again centrifuged. The supernatant is discarded and the remaining pellet frozen at -20°C. In step 2, based on the thus prepared aliquots, the protein extraction is performed with either extraction buffer EB1 or EB2, at either +4°C or +20°C resulting in four different experimental settings (see Table 1). Within each experimental setting four consecutive extractions are performed. All extractions are run for approx. 8-10 hours and are performed in glass vials under gentle stirring.

**Table 1:**

| | | |
|---|---|---|
| 1. | experimental setting 1 | extraction with 10 volumes of EB1 at + 4° C |
| 2. | experimental setting 2 | extraction with 10 volumes of EB1 at + 20° C |
| 3. | experimental setting 3 | extraction with 10 volumes of EB2 at + 4° C |
| 4. | experimental setting 4 | extraction with 10 volumes of EB2 at + 20° C |

### Example 3: Two step extraction of G-CSF from partially permeabilized E. coli cells using NDSB

### Example 3 a)

G-CSF is expressed as described in example 1. A two-step extraction is applied as in example 2. In step 1 cells are permeabilized with 10 volumes of PB2 for approximately 30 minutes. In step 2 all subsequent extractions are performed with 10 volumes of EB3 or EB4 both at +4°C. All extractions are run for approx. 8-10 hours and are performed in glass vials under gentle stirring.

### Example 3 b)

G-CSF is expressed as described in example 1. Then 10 volumes of 0.2%NLS or 0.1%NLS are used at +4°C in step 1, for partial permeabilization, combined with only one extraction in step 2, of approximately 8-10 hours duration, with 25 volumes of EB3.

### Example 3 c)

G-CSF is expressed as described in example 1.10 volumes of 0.05%NLS are used in step 1 for permeabilization (permeabilization is performed as described in example 2) and 20 volumes of 2.0%NDSB-201 in 40mM TRIS/HCl pH 8.0 containing 0.05% NLS in step 2 for extraction. Again extraction is performed as described in example 2

### Example 4: Two step extraction of G-CSF from partially permeabilized E. coli cells using a combination of NLS and NDSB

G-CSF is expressed as described in example 1. Host cells are re-suspended in 10 volumes of permeabilization buffer PB3, e.g., 1g wet pellet in 10ml of buffer. After 40 min incubation at +4°C using gentle mixing, the suspension of cells is centrifuged for 10 minutes at +4°C, 5000 rpm and the pellet is re-suspended in 20 volumes of the extraction buffer EB5.
The cells are suspended over night in extraction solution at +4°C under gentle mixing.

### Example 5: One step extraction of G-CSF using NLS or a combination of NLS and NDSB

G-CSF is expressed as described in example 1. Host cells (1g wet pellet) are re-suspended in minimum 10 ml of extraction solution EB1 or EB5 and extracted over night at +4°C under gentle mixing.

### Example 6: One step extraction of GFP using NLS

Expression of GFP is performed as described in example 1, using the expression plasmid pET19b and strain E. coli BL21 DE3, both from Novagen, Madison USA. GFP is expressed soluble within the host cell. While omitting the permeabilization step, extraction is performed as described in example 2.

### Example 7: Further purification of G-CSF

The resulting suspension of depleted cells and extraction solution containing G-CSF is subjected to either of the following further purification steps:
The suspension is centrifuged and the cells are discarded. The supernatant is subjected to further chromatographic purification.
   Alternatively after the extraction phase is finished, the cell suspension is loaded directly into an expanded bed adsorption (EBA) column to remove the cells and adsorb the target protein onto the matrix in the expanded bed mode, this is followed by further chromatographic purification.

### Example 8: Determination of G-CSF in the extracts

The amount of total G-CSF in the pooled extract is determined densitometrically on commercially purchased SDS-PAGE gels (NuPage 4-12 % Bis-TrisGel, Invitrogen (catalogue 2003, no.NP0321BOX), in the presence of a reducing agent using the NuPage electrophoresis system. SDS-PAGE gels are Coomassie stained in a generally known manner.

### Example 9: Determination of biological activity of G-CSF in the extracts

The biological activity of G-CSF is determined on the NFS-60 cell line according to a published procedure *(*Hammerling U. et al. J. Pharm Biomed Anal 13, 9-20 (1995)) using international standard human recombinant G-CSF 88/502, NIBSC Potters Bar, Hertfordshire, UK.

### ABBREVIATIONS

EBA: Expanded Bed Adsorption
G-CSF: Granulocyte Colony Stimulating Factor
NLS: N-Lauroyl Sarcosine (= "sarcosyl")
NDSB: Non-detergent sulfobetaine

## Claims

1. A process for the recombinant production of a polypeptide of interest within a host cell which comprises a cell wall and at least one cell membrane, comprising
(i) cultivation of said host cell under conditions which cause expression of the polypeptide of interest or of a precursor of the polypeptide of interest in at least partially correctly folded form within said host cell and
(ii) extraction of the polypeptide of interest or the precursor of the polypeptide of interest from said host cell under non denaturing conditions, whereby the cell membrane is partially permeabilized while the cell remains intact and whereby the polypeptide of interest is extracted through the cell membrane and the cell wall.

2. The process according to claim 1, further comprising purification of said protein.

3. The process according to claim 1 or 2, wherein the polypeptide of interest is expressed soluble in the cytoplasm of the host cell.

4. The process according to claim 1 or 2, wherein the polypeptide of interest is expressed within the host cell in form of classical or non-classical inclusion bodies.

5. The process according to any of the preceding claims, wherein the polypeptide of interest is expressed in a bacterial or a yeast host cell.

6. The process according to any of the preceding claims, wherein the polypeptide of interest is expressed in an E. coli host cell.

7. The process according to any of the preceding claims wherein the extraction of the polypeptide of interest is performed using a mild detergent.

8. The process according to claim 7 wherein the mild detergent is NLS.

9. The process according to claim 8 wherein the concentration of NLS is between 0.05% and 0.2%.

10. The process according to any of the preceding claims wherein the extraction of the polypeptide of interest is performed using a mild detergent and in addition an extraction substance.

11. The process according to claim 10 wherein the mild detergent is NLS and the extraction substance is NDSB.

12. The process according to claim 11 wherein NLS is used in a concentration of between 0.05% and 0.1 %, and NDSB is used in a concentration of between 0,2 % and 2%.

13. The process according to any of the preceding claims, wherein the extraction of the polypeptide of interest is performed either by a single extraction cycle or by a series of consecutive extraction cycles.

14. The process according to claim 13, wherein the extraction of the polypeptide of interest is performed by a series of between 1 and 4 consecutive extraction cycles.

15. The process according to claim 13 or 14, wherein extraction of the polypeptide of interest is performed with only a mild detergent and with an extraction substance in addition to a mild detergent in consecutive extraction cycles.

16. The process according to any of the preceding claims wherein prior to extraction of the polypeptide of interest the host cell is subjected to a partial permeabilization step.

17. The process according to claim 16, wherein the host cell is partially permeabilized by incubation in a solution comprising a mild detergent.

18. The process according to claim 17, wherein the mild detergent is NLS.

19. The process according to claim 18, wherein NLS is used in a concentration of between 0.05% to 0.2%.

20. The process according to any of the claims 16 to 19, wherein the extraction of the polypeptide of interest is performed using an extraction substance and no mild detergent.

21. The process according to claim 20, wherein the extraction substance is NDSB.

22. The process according to claim 21, wherein the concentration of NDSB is between 0.2% and 2%.

23. The process according to any of the preceding claims, wherein the polypeptide of interest is G-CSF.

24. Use of NLS for performing the extraction according to any of the preceding claims.

25. Use of NLS as mild detergent for the partial permeabilization of a cell membrane of a host cell in a process according to claims 1 to 23.

26. Use of NDSB as extraction substance in the process according to claims 10 to 23.
